(19) Europäisches Patentamt
European Patent Office
Office européen des brevets

(11) Veröffentlichungsnummer: **0 069 880**
**B1**

(12) # EUROPÄISCHE PATENTSCHRIFT

(45) Veröffentlichungstag der Patentschrift: **12.09.84**

(51) Int. Cl.³: **C 07 C 69/757**, C 07 C 67/333

(21) Anmeldenummer: **82105406.1**

(22) Anmeldetag: **19.06.82**

(54) **Cyclopentanonderivate und Verfahren zu deren Isomerisierung.**

(30) Priorität: **01.07.81 US 279492**

(43) Veröffentlichungstag der Anmeldung: **19.01.83 Patentblatt 83/3**

(45) Bekanntmachung des Hinweises auf die Patenterteilung: **12.09.84 Patentblatt 84/37**

(84) Benannte Vertragsstaaten: **AT BE CH DE FR GB IT LI NL**

(56) Entgegenhaltungen: **DE - A - 2 729 121**

(73) Patentinhaber: **F. HOFFMANN-LA ROCHE & CO. Aktiengesellschaft, CH-4002 Basel (CH)**

(72) Erfinder: **Manchand, Percy, 15 Prescott Avenue, Montclair New Jersey (US)**

(74) Vertreter: **Grossner, Lutz, Dr. et al, Grenzacher Strasse 124 Postfach 3255, CH-4002 Basel (CH)**

EP 0 069 880 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Cyclopentanonderivate der Formel V

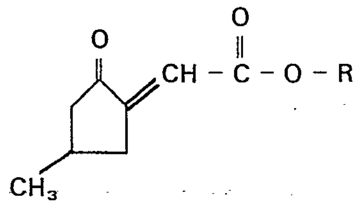

(V)

worin R Wasserstoff oder Niederalkyl ist.

In anderer Hinsicht betrifft die vorliegende Erfindung ein Verfahren zur Umwandlung einer Verbindung der Formel V in eine Verbindung der Formel

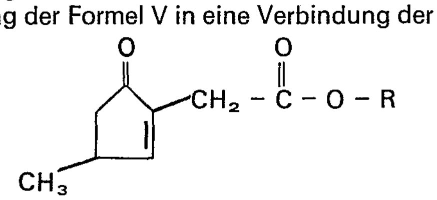

(VI)

worin R die obige Bedeutung hat.

Erfindungsgemäss wird eine Verbindung der Formel V mit einem Übergangsmetall, einem Salz oder Oxid davon und einer Base als Katalysator behandelt, um eine Verbindung der Formel VI zu erhalten.

Der hier verwendete Ausdruck „Niederalkyl" bezieht sich auf geradkettige und verzweigte Alkylgruppen mit 1-7 C-Atomen wie Methyl, Äthyl, Propyl, Butyl und n-Butyl. Bevorzugte Alkylgruppen sind Methyl, Äthyl und n-Butyl.

Die Verbindungen der Formeln V und VI sind als Zwischenprodukte bei der Herstellung von Prostaglandinen wie denen der US-Patentschrift Nr. 4052446 von Wert.

Die Verbindungen der Formel VI können in eine Verbindung der Formel

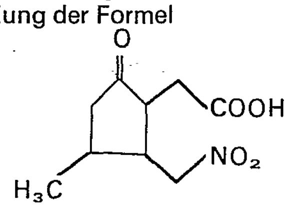

(VIII)

übergeführt werden, die ein bekanntes Zwischenprodukt bei der Herstellung von therapeutisch aktiven Prostaglandinen (US-Patent Nr. 4052446) darstellen.

Das nachfolgende Reaktionsschema beschreibt die vorliegende Erfindung. In dem Reaktionsschema sind die Verfahrensschritte mit 1-6 und die Zwischenprodukte mit I-VIII bezeichnet, wobei R die obige Bedeutung hat. Alle Verbindungen mit einem asymmetrischen C-Atom können in diesen Verfahren als Razemate hergestellt werden. In einer bevorzugten Ausführungsform werden diese Razemate gespalten, um optisch aktive Verbindungen zu erhalten, welche die bevorzugten erfindungsgemässen Verbindungen darstellen. Die Razemate können auf den verschiedenen Stufen des Verfahrens nach an sich bekannten Methoden zur Spaltung einer Säure oder eines Esters gespalten werden. Gewünschtenfalls kann die Säure der Formel VIII beispielsweise gemäss dem in US-Patentschrift Nr. 4154963 beschriebenen Verfahren gespalten werden.

*Reaktionsschema*

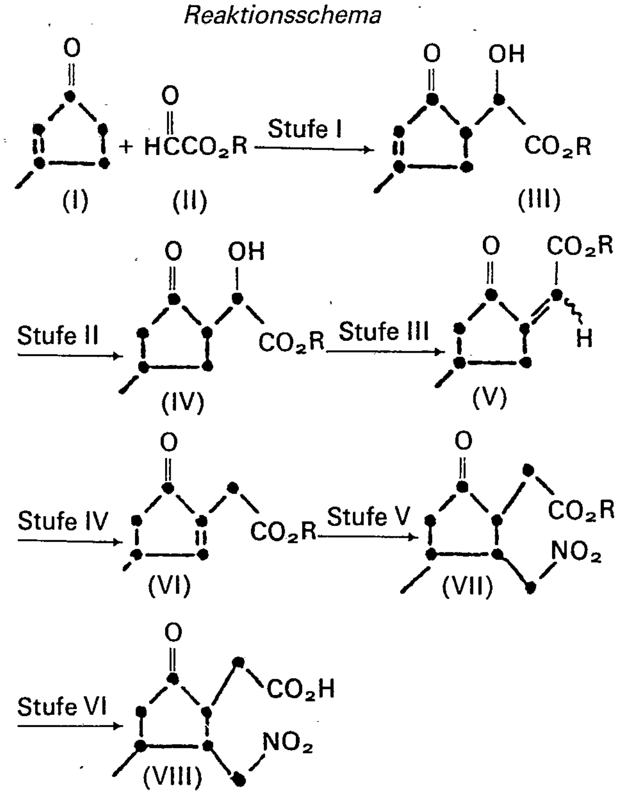


Die Ausgangsstoffe im Schritt 1 des Reaktionsschemas sind 3-Methylcyclopentenon (I) und ein Glyoxylat der Formel II oder eine Verbindung, die in der Lage ist, das Glyoxylat II freizusetzen. Diese Ausgangsstoffe reagieren in einer Aldolkondensation unter Bildung einer Verbindung der Formel III. Bevorzugte Glyoxylate sind Methylglyoxylat, Äthylglyoxylat und n-Butylglyoxylat oder eine Verbindung, die Glyoxylat freisetzt, beispielsweise Polymere der vorerwähnten Glyoxylate. Das Verhältnis der Ausgangsstoffe im Reaktionsgemisch ist nicht kritisch. Vorzugsweise liegt jedoch das Glyoxylat in geringem stöchiometrischen Überschuss vor.

Vorzugsweise wird 3-Methylcyclopentenon mit Äthylglyoxylat in einem Lösungsmittel, das einen geeigneten Katalysator enthält, umgesetzt. Jedes übliche inerte organische Lösungsmittel kann angewandt werden. Beispiele für solche Lösungsmittel sind Toluol, Tetrahydrofuran (THF), Dimethylformamid (DMF), Dimethylsulfoxid (DMSO) und Dimethoxyäthan (DME). Toluol ist bevorzugt. Die Menge des Lösungsmittels ist nicht kritisch, vorzugsweise wird so viel Lösungsmittel verwendet, dass alle Reaktionskomponenten gelöst werden.

Ein geeigneter Katalysator kann jede organische heterocyclische Aminbase sein, wie Morpholin, Piperidin, Pyrrolidin, Pyridin oder Prolin, wobei Morpholin bevorzugt ist. Niedrige Konzentrationen des Katalysators, beispielsweise 0,001 bis 10% (bezogen auf 3-Methylcyclopentenon), sind bevorzugt, insbesondere 0,05 bis 5%, vor allem 3%.

Temperatur und Druck sind in der Reaktion I nicht kritisch, vorzugsweise arbeitet man unter Inertgas und Atmosphärendruck und bei Temperaturen zwischen 20°C und Rückflusstemperatur. Bevorzugt wird die Reaktion beim Siedepunkt des Reaktionsgemisches ausgeführt, um aus polymeren Glyoxylatformen monomere Glyoxylate zu erhalten, wobei hohe Ausbeuten erhalten werden. Das Inertgas kann für diese und alle nachfolgenden Reaktionen jedes gewöhnliche Inertgas, wie Stickstoff oder Argon, sein.

Das Kondensationsprodukt III (wie auch die Produkte in nachfolgenden Reaktionsschritten) kann gewünschtenfalls nach an sich bekannten Methoden wie Destillation oder Chromatographie an Säulen, Papier, Hochdruckflüssigchromatographie oder Gelfiltration isoliert werden.

Stufe II kann mit einer isolierten Verbindung der Formel III ausgeführt werden oder, vorzugsweise, mit dem in Stufe I nach üblichem Eindampfen erhaltenen Reaktionsgemisch. Die ringständige Doppelbindung der Verbindung III wird in an sich bekannter Weise, beispielsweise mittels eines Hydrierkatalysators wie Palladium auf Kohle, hydriert.

In Stufe III wird die Verbindung IV mit einem geeigneten Dehydrierungsmittel zu einer Verbindung der Formel V dehydriert. Die Dehydrierung kann in an sich bekannter Weise vorgenommen werden, beispielsweise durch Zusatz einer Lösung von p-Toluolsulfonsäuremonohydrat in Toluol oder $P_2O_5$-Aluminiumoxid (neutral) in Toluol. Andere bekannte Dehydrierungsmittel, die verwendet werden können, sind $FeCl_3$, $FeCl_3$-Silikagel, $BF_3$, Diäthyläther, MgO, MgO-Molekularsiebe, $P_2O_5$, $P_2O_5$-Silikagel, wasserfreies $P_2O_5$-$Al_2O_3$, $Na_2CO_3$, Acetanhydrid-Essigsäure, $Al_2O_3$, Methansulfochlorid-Pyridin, Natriumacetat, Methansulfochlorid-Triäthylamin und Perchlorsäure. Für diese Hydrierungsmittel können die bekannten Reaktionsbedingungen angewandt werden.

In Stufe IV wird die Verbindung der Formel V zu einer Verbindung der Formel VI isomerisiert. Diese Isomerisierung kann in einem inerten organischen Lösungsmittel unter Verwendung eines Übergangsmetalls oder eines Salzes oder Oxids davon als Katalysator ausgeführt werden. Vorzugsweise wird die Reaktion bei erhöhten Temperaturen, insbesondere beim Siedepunkt des Lösungsmittels, durchgeführt. Als Lösungsmittel können niedere Alkanole angewandt werden. Bevorzugt sind geradkettige oder verzweigte niedere Alkanole mit 1-7 C-Atomen wie Methylalkohol, Äthylalkohol, Butylalkohol und Propylalkohol. Äthanol ist besonders bevorzugt.

Die Isomerisierung der Doppelbindung in der Verbindung der Formel V kann auch bewerkstelligt werden, indem anstelle des Übergangsmetalls oder des Salzes oder Oxids davon eine anorganische oder organische Base als Katalysator eingesetzt wird. Beispielsweise kann die Isomerisierung mit einem organischen Amin wie Pyridin als Katalysator und Lösungsmittel durchgeführt werden, oder unter Verwendung eines Alkylamins wie Dimethylaminopyridin und einem Lösungsmittel wie Toluol oder Hexan. Bevorzugte Reaktionstemperaturen sind 50-130°C.

Jedes Übergangsmetall kann in reiner Form oder als Salz in hydrierter oder dehydrierter Form in der Stufe IV als Katalysator verwendet werden, beispielsweise $RhCl_33H_2O$, $PdCl_2$, Rh, Pd, $PdCl_2(C_6H_5CN)_2$, Rh auf Graphit, oder ZnO. Der bevorzugte Übergangsmetallkatalysator ist $RhCl_33H_2O$. Vorzugsweise wird der Katalysator in Mengen von 5-20% bezogen auf das Lösungsmittel eingesetzt. Es wurde überraschend gefunden, dass die Verwendung des Katalysators die Bildung unerwünschter Isomere und die Reaktionszeit vermindert. Durch dieses Verfahren wurde die Verbindung der Formel VI ohne wesentliche Bildung anderer Isomere erhalten, wenn die Katalysatorkonzentration 5% oder mehr bezogen auf das Gewicht des Lösungsmittels betrug. Die Anwendung von weniger Katalysator erfordert verlängerte Reaktionszeiten (3-4 Tage) und führte zur Bildung der Verbindung der Formel VI als Gemisch mit anderen verwandten Isomeren.

In einer bevorzugten Ausführungsform wird in Stufe IV ein organisches Amin als Katalysator verwendet, weil eine solche Verbindung im Gebrauch billiger ist als ein Übergangsmetall.

Die Verbindung der Formel VII kann in Stufe V durch Behandlung der Verbindung VI mit Nitromethan in Gegenwart einer Base erhalten werden. Hierfür kann jede gewöhnliche Base angewandt werden, bevorzugt sind niedere Alkoxide insbesondere Alkalimetall-nieder-Alkoxide und Amine, insbesondere tertiäre und quaternäre Amine und Pyridin. Eine besonders bevorzugte Base ist Benzyltrimethylammoniumhydroxid. Temperatur und Druck sind nicht kritisch, vorzugsweise arbeitet man unter Inertgasatmosphäre wie Stickstoff und bei erhöhten Temperaturen z.B. 65-70°C.

Die Verbindung der Formel VIII, 2α-Carboxymethyl-3β-nitromethyl-4α-methylcyclopentan-1-on, ist eine bekannte Verbindung, die z.B. in US-Patent Nr. 4154963 beschrieben ist. Sie kann in Stufe VI durch Hydrolyse einer Verbindung der Formel VII erhalten werden. Hierfür kann die isolierte Verbindung der Formel VII oder vorzugsweise das in Stufe V nach Eindampfen erhaltene Reaktionsgemisch eingesetzt werden. Für die Hydrolyse der Verbindung der Formel VII können die üblichen Hydrolysemethoden angewandt werden. Vorzugsweise verwendet man verdünnte wässerige Mineralsäure wie Schwefelsäure oder verdünntes wässeriges Alkalimetallhydroxid. Hydrolyse mit verdünnter Natriumhydroxidlösung ist besonders bevorzugt. Temperatur und Druck sind nicht kritisch, vorzugsweise arbeitet man bei Atmosphärendruck und unter Stickstoff bei leicht erhöhten Temperaturen bis etwa 40°C. Die Verbindung der Formel VIII kann dann durch übliche Extraktions- und Filtrationsmethoden erhalten werden.

Erhaltene Razemate können in die optisch aktiven Komponenten nach an sich bekannten Spaltungsmethoden gespalten werden. Säuren können mit optisch aktiven Basen behandelt werden, um diastereomere Salze zu erhalten, die dann durch Kristallisation getrennt werden können.

Die Erfindung wird durch die nachfolgenden Beispiele weiter erläutert.

*Beispiel 1*

62,9 g α-Hydroxy-4-methyl-2-oxo-pentan-essigsäure-äthylester wurde in 940 ml Toluol mit 3 g p-Toluolsulfonsäuremonohydrat 2 Stunden bei 80°C gerührt und dann weitere 2 Stunden bei 85°C unter azeotroper Entfernung des Wassers. Das Gemisch wurde auf Raumtemperatur gekühlt, nacheinander mit 250 ml Kochsalzlösung und 250 ml Wasser gewaschen, über Magnesiumsulfat getrocknet und eingedampft. Man erhielt 62 g eines dunklen rötlichbraunen Öls, das nach Destillation ein hellgelbes homogenes Öl, Siedepunkt 68-88°C/0,05 mm Hg, lieferte und das als 4-Methyl-2-oxo-cyclopentyliden-essigsäure-äthylester identifiziert wurde.

*Beispiel 2*

Ein Gemisch von 154,8 g α-Hydroxy-4-methyl-3-oxo-cyclopentan-essigsäure-butylester, 2,3 l Toluol und 12,9 g p-Toluolsulfonsäure-monohydrat wurde 6,5 Stunden unter Stickstoff bei 75-78°C gerührt. Danach wurde das Reaktionsgemisch auf 15°C gekühlt, mit 3 1-l-Portionen Wasser gewaschen, über Magnesiumsulfat getrocknet und eingedampft. Man erhielt 153 g 4-Methyl-3-oxo-cyclopentyliden-essigsäure-butylester vom Siedepunkt 82-84°C/0,05 mm Hg als rotes Öl.

*Beispiel 3*

Ein Gemisch von 40,1 g 4-Methyl-2-oxo-cyclopentyliden-essigsäure-butylester, 500 ml 90%iges wässeriges Äthanol und 2 g Rhodiumtrichloridtrihydrat wurde 14 Stunden unter Stickstoff zum Rückfluss erhitzt und dann unter vermindertem Druck eingedampft. Der Rückstand wurde mit 200 ml gesättigter Kochsalzlösung versetzt, das Gemisch 15 Minuten gerührt und mit 200-ml-Portionen Diäthyläther extrahiert. Der Extrakt wurde über Magnesiumsulfat getrocknet und das Filtrat mit 100 g Aluminiumoxid (I) aufgeschlämmt. Das Aluminiumoxid wurde abfiltriert und mit 400 ml Äther gewaschen. Filtrat und Waschwasser wurden eingedampft und lieferten 31 g (77%) 3-Methyl-5-oxo-1-cyclopenten-1-essigsäure-äthylester als farbloses Öl, Siedepunkt 90°C/0,05 mm Hg. Das Produkt ist relativ unstabil und sollte unter Stickstoff eingefroren aufbewahrt werden.

*Beispiel 4*

Ein Gemisch von 103,8 g 4-Methyl-2-oxo-cyclopentyliden-essigsäure-butylester, 1,25 l 30%iges wässeriges Äthanol und 5,19 g Rhodiumtrichloridtrihydrat wurde unter Rühren und unter Stickstoff 24 Stunden zum Rückfluss erhitzt, auf 40°C gekühlt und zu einem viskosen roten Öl eingedampft. Das Öl wurde mit 1 l 20%iger Kochsalzlösung versetzt und die Lösung mit 3mal 600 ml Toluol extrahiert. Die vereinigten Extrakte wurden über Magnesiumsulfat getrocknet, auf 500 ml eingedampft und mit 200 g neutralem Aluminiumoxid (I) aufgeschlämmt. Das Aluminiumoxid wurde abfiltriert und 3mal mit 250 ml Toluol gewaschen. Filtrat und Waschwasser wurden eingedampft und lieferten 68 g (83%) 3-Methyl-5-oxo-1-cyclopenten-1-essigsäure-äthylester als hellgelbes Öl, Siedepunkt 97°C/0,05 mm Hg. Das Produkt ist relativ unstabil und sollte unter Stickstoff eingefroren werden.

*Beispiel 5*

1. Eine Lösung von 10 g des in Beispiel 1 erhaltenen Produktes wurde in 100 ml Pyridin 23,5 Stunden unter Argonatmosphäre bei 96°C gerührt. Das Lösungsmittel wurde bei 45°C unter vermindertem Druck entfernt, wobei 10 g 3-Methyl-5-oxo-1-cyclopenten-1-essigsäure-äthylester mit einer Reinheit von 96% (Hochdruckflüssigchromatographie) erhalten wurden.

2. Eine Lösung von 1 g des gemäss Beispiel 1 erhaltenen Produktes wurde in 15 ml Toluol, die 100 mg 4-Dimethylamonopyridin enthielten, 20 Stunden zum Rückfluss erhitzt. Das erhaltene Gemisch wurde auf Raumtemperatur abgekühlt, mit 15 ml 1 N Salzsäure und danach 2mal mit Wasser gewaschen. Die organische Phase wurde über Magnesiumsulfat getrocknet und eingedampft und lieferte 960 mg 3-Methyl-5-oxo-1-cyclopenten-1-essigsäure-äthylester.

Die nachfolgenden Beispiele A-D beziehen sich auf die Herstellung des als Ausgangsmaterial in den Beispielen 1 und 2 verwendeten Äthyl- bzw. Butylesters.

*Beispiel A*

Ein Gemisch von 48 g frisch destilliertem 3-Methyl-2-cyclopenten-1-on, 66,4 g frisch destilliertem Äthylglyoxylat, 1,31 g Morpholin und 650 ml Toluol wurde 21 Stunden zum Rückfluss erhitzt, auf Raumtemperatur abgekühlt und eingedampft. Das so erhaltene Rohmaterial wurde direkt in der Hydrierung von Beispiel C eingesetzt. Es enthält α-Hydroxy-4-methyl-2-oxo-3-cyclopenten-1-yl-essigsäure-äthylester, der aus dem Rohprodukt durch Destillation als viskoses Öl vom Siedepunkt 150°C/10 mm Hg erhalten werden kann.

*Beispiel B*

Ein Gemisch von 96,1 g frisch destilliertem 3-Methyl-2-cyclopenten-1-on, 169 g frisch destilliertem n-Butylglyoxylat, 1,3 l Toluol und 2,61 g Morpholin wurde 24 Stunden zum Rückfluss erhitzt und dann unter vermindertem Druck eingedampft. Man erhielt 263 g rohen razemischen α-Hydroxy-4-methyl-3-oxo-3-cyclopenten-1-essigsäure-butylester als viskoses hellbraunes Öl, Siedepunkt 130°C/0,05 mm Hg, das in Beispiel D eingesetzt wird.

*Beispiel C*

99,1 g roher α-Hydroxy-4-methyl-2-oxo-3-cyclopenten-1-yl-essigsäure-äthylester wurden in 882 ml Toluol über 9,91 g (10%) Palladium/-Kohle-Katalysator bei Raumtemperatur und einem Druck von 175-350 kPa hydriert. Nachdem die Wasserstoffaufnahme aufgehört hatte, wurde der Katalysator abfiltriert, das Lösungsmittel abgedampft, und man erhielt 101 g eines viskosen amberfarbenen Öls. Das Öl wurde destilliert und lieferte α-Hydroxy-4-methyl-2-oxo-cyclopentan-essigsäure-äthylester, Siedepunkt 96-111°C/0,05 mm Hg.

*Beispiel D*

226 g α-Hydroxy-4-methyl-3-oxo-3-cyclopenten-1-essigsäure-butylester wurden in 2 l Toluol über 22,6 g Pd/C-Katalysator bei Raumtemperatur und einem Druck von 175-350 kPa hydriert. Der Katalysator wurde abfiltriert und das Lösungsmittel eingedampft und man erhielt 251 g eines rotbraunen viskosen Öls, das nach Destillation razemisches α-Hydroxy-4-methyl-2-oxo-cyclopentan-essigsäure-butylester vom Siedepunkt 110-117°C/0,05 mm Hg lieferte.

Die nachfolgenden Beispiele E und F beschreiben die Umwandlung einer Verbindung der Formel VI in die Verbindung VIII.

*Beispiel E*

Ein Gemisch von 30,4 g 3-Methyl-5-oxo-1-cyclopenten-1-essigsäure-äthylester, 122 ml Nitromethan und 11,7 g Triton B wurde 2 Stunden unter Stickstoff bei 65-70°C gerührt. Nach Kühlen auf 10°C und Zusatz von 23 ml kalter (10°C) 4N Schwefelsäure wurde weitere 30 Minuten gerührt. Das Gemisch wurde in 115 ml gesättigte Kochsalzlösung gegossen und mit 230 ml Äther extrahiert. Der Extrakt wurde mit Kochsalzlösung neutral gewaschen, über Magnesiumsulfat getrocknet und eingedampft und lieferte 43 g rohen 3-Methyl-2-(nitromethyl)-5-oxo-cyclopentan-essigsäure-äthylester, der mit 355 ml 1N Natronlauge 30 Minuten unter Stickstoff bei 40°C gerührt wurde. Das Gemisch wurde auf Raumtemperatur abgekühlt und mit 150 ml Methylenchlorid extrahiert. Die wässerige Phase wurde auf 10°C gekühlt, mit 365 ml kalter (10°C) 1N Salzsäure angesäuert, mit 3mal 200 ml Äthylacetat extrahiert und über Magnesiumsulfat getrocknet. Nach Zusatz von 10 g Aktivkohle wurde das Gemisch einige Minuten gerührt und über Diatomeenerde filtriert. Eindampfen des Filtrats unter vermindertem Druck bei Temperaturen unter 30°C lieferte 37,4 g 4α-Methyl-3β-nitromethyl-2α-carboxymethyl-cyclopentanon in Form hellgelber Kristalle, die nach Umkristallisation aus 90 ml heissem Äthylacetat/Hexan 23 g (64%) 4α-Methyl-3β-nitromethyl-2α-carboxymethyl-cyclopentanon vom Schmelzpunkt 113-114°C lieferten.

*Beispiel F*

Zu 4,3 g 4α-Methyl-3β-nitromethyl-2α-carboxymethyl-cyclopentanon in 60 ml Methylenchlorid wurden 7 g Strychnin unter Stickstoffatmosphäre gegeben. Nach 10 Minuten Rühren entstand eine homogene Lösung. Das Gemisch wurde weitere 2 Stunden unter Stickstoff gerührt und unter vermindertem Druck bei einer Badtemperatur unterhalb 30°C eingedampft. Der erhaltene Gummi (der 5-10 ml Methylenchlorid enthielt) wurde mit 60 ml Aceton versetzt und das verbleibende Methylenchlorid wurde unter vermindertem Druck abgedampft. Das Gemisch wurde bei Raumtemperatur 30 Minuten lang kräftig gerührt und das Salz der nicht erwünschten (+)-Säure abfiltriert. Es wurde mit 2mal 30 ml Aceton gewaschen. Filtrat und Waschwasser wurden unter vermindertem Druck (bei Temperatur unterhalb 30°C) eingedampft und lieferten 5,3 g eines Gummis, der in 100 ml Äthylacetat gelöst wurde. Unlösliches wurde abfiltriert und mit 4mal 100 ml kalter (15°C) Salzsäure gewaschen. Die wässerigen Waschflüssigkeiten wurden mit 100 ml Äthylacetat rückextrahiert und die vereinigten Extrakte mit 2mal 100 ml Wasser neutral gewaschen, über Magnesiumsulfat getrocknet und unter vermindertem Druck bei Temperaturen unterhalb 30°C zu einem Öl eingedampft. Das Öl wurde in 5 ml Äthylacetat gelöst und die Lösung mit 5 ml Hexan versetzt. Die Lösung wurde bei −10°C über Nacht stehengelassen und die kristalline razemische Säure (470 mg) abfiltriert. Eindampfen des Filtrats unter vermindertem Druck (Temperatur unterhalb 30°C) lieferte ein Öl, das unter Hochvakuum (0,1 mm/25°C) über Nacht aufbewahrt wurde und 1,7 g (79,5%) [1R-(1α-2β-3α)]-2-(Nitromethyl)-3-methyl-5-oxo-1-cyclopentan-essigsäure lieferte. Dieses Rohmaterial wurde direkt für die Reduktion und Lactonisierung zu 3,3AR,4,5,6,6AS-Hexahydro-4S-nitromethyl-5R-methyl-2H-cyclopenta[b]-furan-on eingesetzt.

Die oben beschriebene rohe Säure wurde aus Hexan kristallisiert und lieferte [1R-(1α-2β-3α)]-2-(Nitromethyl)-3-methyl-5-oxo-1-cyclopentan-essigsäure vom Schmelzpunkt 56-58°C.

**Patentansprüche** für die Vertragsstaaten BE, CH, DE, FR, GB, IT, LI, NL

1. Eine Verbindung der Formel

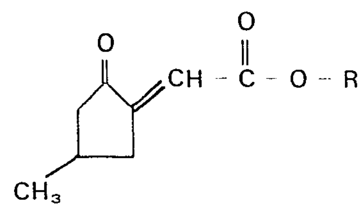

(V)

worin R Wasserstoff oder Niederalkyl ist.

2. Verbindung gemäss Anspruch 1, in der R Methyl, Äthyl oder n-Butyl ist.

3. 4-Methyl-2-oxo-cyclopentyliden-essigsäure-äthylester.

4. 4-Methyl-2-oxo-cyclopentyliden-essigsäure-butylester.

5. Verfahren zur Herstellung einer Verbindung der Formel

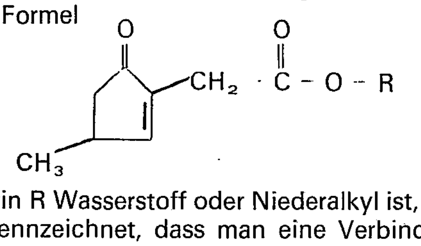

(VI)

worin R Wasserstoff oder Niederalkyl ist, dadurch gekennzeichnet, dass man eine Verbindung der Formel

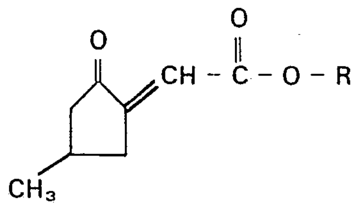
(V)

worin R die obige Bedeutung hat, mit einem Übergangsmetall, einem Salz oder Oxid davon oder einer Base als Katalysator behandelt.

6. Verfahren nach Anspruch 5, dadurch gekennzeichnet, dass der Katalysator eine organische Base ist.

7. Verfahren nach Anspruch 6, dadurch gekennzeichnet, dass die organische Base Pyridin oder Dimethylaminopyridin ist.

**Patentansprüche** für den Vertragsstaat: AT

1. Verfahren zur Herstellung einer Verbindung der Formel

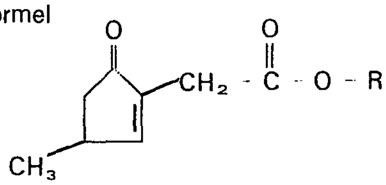
(VI)

worin R Wasserstoff oder Niederalkyl ist, dadurch gekennzeichnet, dass man eine Verbindung der Formel

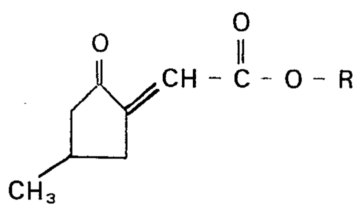
(V)

worin R die obige Bedeutung hat, mit einem Übergangsmetall, einem Salz oder Oxid davon oder einer Base als Katalysator behandelt.

2. Verfahren nach Anspruch 1, dadurch gekennzeichnet, dass der Katalysator eine organische Base ist.

3. Verfahren nach Anspruch 2, dadurch gekennzeichnet, dass die organische Base Pyridin oder Dimethylaminopyridin ist.

**Claims** for contracting States BE, CH, DE, FR, GB, IT, LI, NL

1. A compound of the formula

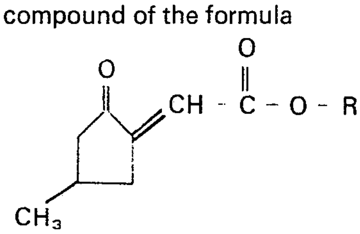
(V)

where R is hydrogen or lower alkyl.

2. The compound of Claim 1 wherein R is methyl, ethyl or n-butyl.

3. 4-Methyl-2-oxo-cyclopentylidene acetic acid ethyl ester.

4. 4-Methyl-2-oxo-cyclopentylidene acetic acid butyl ester.

5. A process for producing a compound of the formula

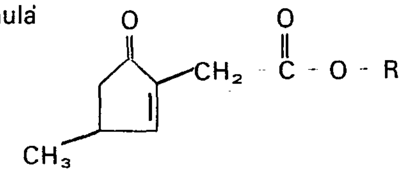
(VI)

wherein R is hydrogen or lower alkyl, characterized in that a compound of the formula

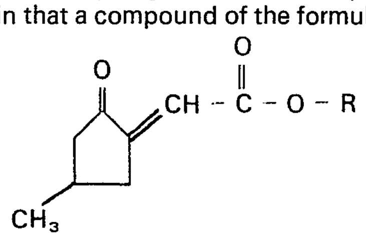
(V)

wherein R is as defined above, is treated with a transition metal, salt or oxide thereof or a base as catalyst.

6. A process as in Claim 5 wherein the catalyst is an organic base.

7. A process as in Claim 6 wherein the organic base is pyridine or dimethylamino pyridine.

**Claims** for contracting State: AT

1. A process for producing a compound of formula

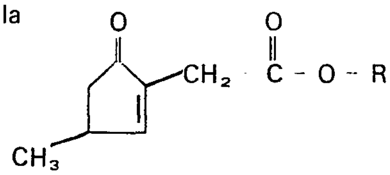
(VI)

wherein R is hydrogen or lower alkyl, characterized in that a compound of the formula

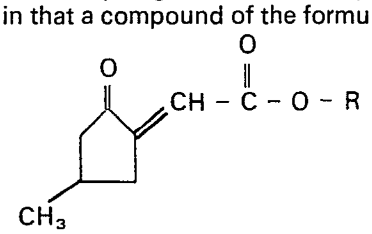
(V)

wherein R is as defined above, is treated with a transition metal, salt or oxide thereof or a base as catalyst.

2. A process as in Claim 1 wherein the catalyst is an organic base.

3. A process as in Claim 2 wherein the organic base is pyridine or dimethylamino pyridine.

**Revendications** pour les Etats contractants CH, DE, FR, GB, IT, LI, NL

1. Composé de formule

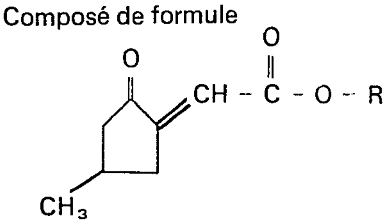
(V)

dans laquelle R représente l'hydrogène ou un groupe alkyle inférieur.

2. Composé selon la revendication 1, dans lequel R représente un groupe méthyle, éthyle ou n-butyle.

3. Le 4-méthyl-2-oxo-cyclopentylidène-acétate d'éthyle.

4. Le 4-méthyl-2-oxo-cyclopentylidène-acétate de butyle.

5. Procédé de préparation d'un composé de formule

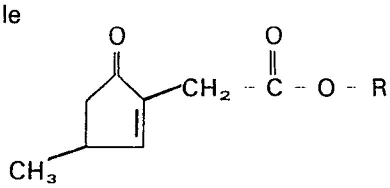

(VI)

dans laquelle R représente l'hydrogène ou un groupe alkyle inférieur, caractérisé en ce que l'on traite un composé de formule

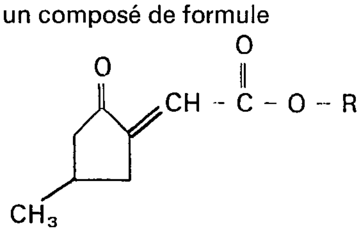

(V)

dans laquelle R a la signification indiquée ci-dessus, par un métal de transition, un sel ou un oxyde d'un tel méta!, ou une base servant de catalyseur.

6. Procédé selon la revendication 5, caractérisé en ce que le catalyseur est une base organique.

7. Procédé selon la revendication 6, caractérisé en ce que la base organique est la pyridine ou la diméthylaminopyridine.

**Revendications** pour l'Etat contractant: AT

1. Procédé de préparation d'un composé de formule

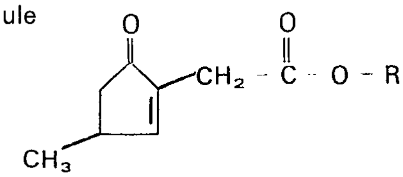

(VI)

dans laquelle R représente l'hydrogène ou un groupe alkyle inférieur, caractérisé en ce que l'on traite un composé de formule

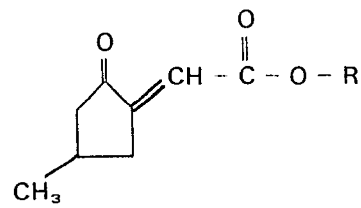

(V)

dans laquelle R a la signification indiquée ci-dessus, par un métal de transition, un sel ou un oxyde d'un tel métal, ou une base servant de catalyseur.

2. Procédé selon la revendication 1, caractérisé en ce que le catalyseur est une base organique.

3. Procédé selon la revendication 2, caractérisé en ce que la base organique est la pyridine ou la diméthylaminopyridine.